# EUROPEAN PATENT SPECIFICATION

(11) **EP 4 164 639 B1**
(45) Date of publication and mention of the grant of the patent: **09.12.2026**
(21) Application number: 21742945.5
(22) Date of filing: 10.06.2021
(51) Int. Cl.: A61K 31/437, A61K 31/4706, A61K 31/573, A61K 45/06, A61P 37/02

(54) **AFIMETORAN IN COMBINATION WITH PREDNISOLONE OR HYDROXYCHLOROQUINE FOR TREATING CUTANEOUS LUPUS ERYTHEMATOSUS**
AFIMETORAN IN KOMBINATION MIT PREDNISOLON ODER HYDROXYCHLOROQUIN ZUR BEHANDLUNG VON HAUTLUPUS ERYTHEMATODES
AFIMETORAN EN COMBINAISON AVEC DE LA PREDNISOLONE OU DE L'HYDROXYCHLOROQUINE POUR LE TRAITEMENT DU LUPUS ÉRYTHÉMATEUX CUTANÉ

(30) Priority: 11.06.2020 IN 202011024586
(43) Date of publication of application: 19.04.2023
(73) Proprietor: Bristol-Myers Squibb Company, Princeton, NJ 08543 (US)
(72) Inventor: SCHIEVEN, Gary L., Lawrenceville, NJ 08648 (US); DUDHGAONKAR, Shailesh, Bangalore Karnataka 560 099 (IN)
(74) Representative: Mathys & Squire
(86) International application number: PCT/US2021/036740
(87) International publication number: WO 2021/252718

(56) References cited:
- WO-A1-2018/005586
- TEBBE B ET AL: "HEUTIGE THERAPEUTISCHE ASPEKTE BEIM KUTANENLUPUS ERYTHEMATODES THERAPEUTIC ASPECTS OF CUTANEOUS LUPUS ERYTHEMATOSUS", DERMATOLOGISCHE MONATSSCHRIFT, JOHANN AMBRISIUS BARTZ, LEIPZIG, DE, DE, vol. 178, no. 3, 1 January 1992 (1992-01-01), pages 99 - 104, XP001033514, ISSN: 0011-9083

## Description

The present invention generally relates to a method of treating a patient having cutaneous lupus erythematosus, comprising administering to said patient a therapeutically effective dose of a TLR7 inhibitor or a pharmaceutically acceptable salt thereof, in combination with a therapeutically effective dose of a second agent selected from prednisolone and hydroxychloroquine, or a pharmaceutically acceptable salt thereof

### BACKGROUND

Toll-like receptors (TLRs) are a family of pattern recognition receptors that recognize highly conserved components of diverse pathogens and induce innate and adaptive immune responses (Akira S, Takeda K, T. Kaisho, T., Nat Immunol. 2001;2:675-680; Pandey S, Agrawal DK., Immunol Cell Biol. 2006;84:333-341; Kawai T, Akira S. J Biochem. 2007;141:137-145). TLR7 and TLR8 have been shown to be expressed in endosomal compartments and to recognize ssRNA molecular patterns. TLR7 is expressed in B cells and plasmacytoid dendritic cells (pDC); TLR8 is expressed in monocytes and myeloid dendritic cells (mDC) (Chuang TH, Ulevitch RJ.. Eur Cytokine Net. 2000;11:372-378; Iwasaki A, Medzhitov R. Nat Immunol. 2004;5:987-995).

Cutaneous lupus erythematosus (CLE) is a skin disorder which can occur as isolated skin manifestation or with concomitant systemic lupus erythematosus (SLE) (Okon LG, Werth VP. Best Pract Res Clin Rheumatol. 2013;27:391-404; Cohen MR, Crosby D. J Rheumatol. 1994;21:1665-1669).

CLE is often associated with the presence of autoantibodies with varying frequency. Similar to SLE, Type I Interferon (IFN) dysregulation is proposed to be a key pathogenic driver in CLE. CLE patients often present with IFN signature in blood and in skin lesions, with blood IFN signature correlating with skin disease activity score (Braunstein I, Klein R, Okawa J, et al., Br J Dermatol. 2012;166:971-975; Meller S, Winterberg F, Gilliet M, et al., Arthritis Rheum. 2005;52:1504-1516, Wenzel J, Zahn S, Mikus S, et al., Br J Dermatol. 2007;157:752-757).

Treatment with sifalimumab (anti-IFNα mAb) and anifrolumab (anti-IFNAR mAb) showed an improvement of skin disease activity scores in SLE patients presenting with cutaneous disease in Phase II clinical trials further supporting the contribution of Type I IFN to the pathogenesis of CLE (Furie R, Khamashta M, Merrill JT, et al. Arthritis Rheumatol. 2017;69:376-86; Petri M, Wallace DJ, Spindler A, et al. Arthritis Rheum. 2013;65:1011-1021). Plasmacytoid dendritic cells (pDCs) are specialized cells that accumulate in the skin of CLE patients and account for 5-10% of the immune infiltrate and have been proposed to be the major source of Type I IFN in CLE skin lesions. The pDCs robustly secrete IFNα in response to TLR7 and TLR9 stimulation by nucleic acid ligands, while TLR8 induces a variety of myeloid cells to produce proinflammatory cytokines such as TNFα and IL-6 (Siegal FP, Kadowaki N, Shodell M, et al. Science 1999;284: 1835-1837; Tomasini D, Mentzel T, Hantschke M, et al., J Cutan Pathol. 2010;37:1132-1139; Vermi W, Lonardi S, Morassi M, et al., Immunobiology 2009;214:877-86).

Apoptotic debris induced by ultra-violet radiation, a well-known environmental trigger of CLE, leads to the accumulation of ribonucleic acid (RNA) and deoxyribonucleic acid (DNA) fragments that would activate TLR7 and TLR9 respectively, leading to pDC activation and Type I IFN production (Wenzel J, Proelss J, Wiechert A, et al., J Am Acad Dermatol. 2007;56: 648-650). Examples of inhibitors of TLR7 are described in Tebbe B, et al. Dermatologische Monatsschrift, 1992; 178:99-104. Examples of inhibitors of TLR7, TLR8 and/or TLR9 are described in International Patent Publication WO 2018/005586 A1.

New methods of treating CLE are desired.

Disclosed herein are methods of treating cutaneous lupus erythematosus, comprising administering to a patient a therapeutically effective dose of a TLR7 inhibitor or a pharmaceutically acceptable salt thereof, in combination with a therapeutically effective dose of prednisolone or a pharmaceutically acceptable salt thereof.

Also disclosed herein are methods of treating cutaneous lupus erythematosus, comprising administering to a patient a therapeutically effective dose of a TLR7 inhibitor or a pharmaceutically acceptable salt thereof, in combination with a therapeutically effective dose of hydroxychloroquine or a pharmaceutically acceptable salt thereof.

### SUMMARY

The present disclosure relates to methods of treating a patient having cutaneous lupus erythematosus, comprising administering to said patient a therapeutically effective dose of a TLR7 inhibitor or a pharmaceutically acceptable salt thereof, in combination with a therapeutically effective dose of a second agent selected from prednisolone and hydroxychloroquine, or a pharmaceutically acceptable salt thereof.

The present invention provides a TLR7 inhibitor or a pharmaceutically acceptable salt thereof for use in a method of treating a patient having cutaneous lupus erythematosus, the method comprising administering a therapeutically effective dose of said TLR7 inhibitor or pharmaceutically acceptable salt thereof, in combination with a therapeutically effective dose of a second agent selected from prednisolone and hydroxychloroquine, or a pharmaceutically acceptable salt thereof, wherein said TLR7 inhibitor is: 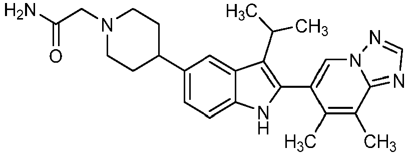 or a pharmaceutically acceptable salt thereof.

In embodiments, said therapeutically effective dose of said TLR7 inhibitor is from 0.1 to 100 mg per day. In embodiments, said therapeutically effective dose of said prednisolone is 0.5 to 50 mg per day. In embodiments, said therapeutically effective dose of said hydroxychloroquine is 1 to 20 mg per day.

In embodiments, said TLR7 inhibitor and said second agent are administered concurrently, or sequentially. In embodiments, said TLR7 inhibitor is administered prior to administration of said second agent. In embodiments, said second agent is administered prior to administration of said TLR7 inhibitor.

The present invention also provides a TLR7 inhibitor or a pharmaceutically acceptable salt thereof, in combination with a second agent selected from prednisolone, or a pharmaceutically acceptable salt thereof, for use in a method of treating a patient having cutaneous lupus erythematosus, wherein said TLR7 inhibitor is:

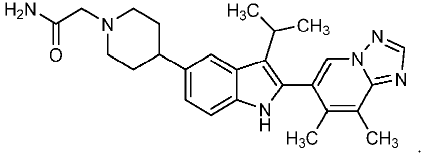

The present invention further provides a TLR7 inhibitor or a pharmaceutically acceptable salt thereof, in combination with a second agent selected from hydroxychloroquine, or a pharmaceutically acceptable salt thereof, for use in a method of treating a patient having cutaneous lupus erythematosus, wherein said TLR7 inhibitor is:

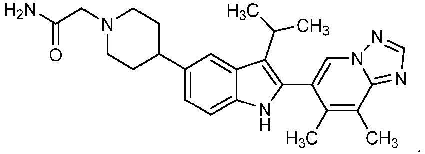

These and other features of the invention will be set forth in expanded form as the disclosure continues.

### BRIEF DESCRIPTION OF THE DRAWINGS

The disclosure and invention is illustrated by reference to the accompanying drawings described below.
FIG. 1 shows the inhibition of cutaneous lesions following treatment with either Compound (I) or prednisolone in female MRL/lpr mice. A: Neck lesions; B: Head lesions; C: Face lesions; D: Ear lesions; E: Total lesions.
FIG. 2 shows the reduction of erythema of cutaneous lesions treatment with either Compound (I) or prednisolone in female MRL/lpr mice. A: Neck Erythema; B: Ear Erythema; C: Total Erythema.
FIG 3 shows the inhibition of circulating antibody titers including anti-dsDNA, anti-smRNP and anti-Ro antibody titers following treatment with either Compound (I) or prednisolone.
FIG 4 shows reduction in the proteinuria and urinary NGAL following treatment with either Compound (I) or prednisolone.
FIG 5 shows Compound (I) inhibits TLR7-induced IL-6 in an ex vivo settings following treatment with either Compound (I) or prednisolone.
FIG 6 shows the total lesions for the female MRL/lpr mice in established disease treated with vehicle, Compound (I), prednisolone, hydroxychloroquine, Compound (I) + prednisolone, and Compound (I) + hydroxychloroquine.
FIG 7 shows the neck erythema score for female MRL/lpr mice treated with vehicle, Compound (I), prednisolone, hydroxychloroquine, Compound (I) + prednisolone, and Compound (I) + hydroxychloroquine.
FIG 8 shows the ear erythema score for female MRL/lpr mice in established disease treated with vehicle, Compound (I), prednisolone, hydroxychloroquine, Compound (I) + prednisolone, and Compound (I) + hydroxychloroquine.
FIG 9 shows the total erythema score for female MRL/lpr mice in established disease treated with vehicle, Compound (I), prednisolone, hydroxychloroquine, Compound (I) + prednisolone, and Compound (I) + hydroxychloroquine.
FIG 10 shows the proteinuria levels for female MRL/lpr mice in established disease treated with vehicle, Compound (I), prednisolone, hydroxychloroquine, Compound (I) + prednisolone, and Compound (I) + hydroxychloroquine.
FIG 11 shows the urinary NGAL levels for female MRL/lpr mice in established disease treated with vehicle, Compound (I), prednisolone, hydroxychloroquine, Compound (I) + prednisolone, and Compound (I) + hydroxychloroquine.
FIG 12 shows the total lesions for the female MRL/lpr mice in advanced disease treated with vehicle, Compound (I), prednisolone, hydroxychloroquine, Compound (I) + prednisolone, and Compound (I) + hydroxychloroquine.
FIG 13 shows the neck erythema score for female MRL/lpr mice treated with vehicle, Compound (I), prednisolone, and Compound (I) + prednisolone.
FIG 14 shows the neck erythema score for female MRL/lpr mice treated with vehicle, hydroxychloroquine, and Compound (I) + hydroxychloroquine.
FIG 15 shows the ear erythema score for female MRL/lpr mice in advanced disease treated with vehicle, Compound (I), prednisolone, hydroxychloroquine, Compound (I) + prednisolone, and Compound (I) + hydroxychloroquine.
FIG 16 shows the total erythema score for female MRL/lpr mice in advanced disease treated with vehicle, Compound (I), prednisolone, hydroxychloroquine, Compound (I) + prednisolone, and Compound (I) + hydroxychloroquine.

### DEFINITIONS

In order that the present description may be more readily understood, certain terms are first defined. Additional definitions are set forth throughout the detailed description.

A "TR7 inhibitor" inhibits the function of TLR7. TLR7 inhibitors can associate with TLR7 reversibly or irreversibly, and include antibodies, small molecules, and millimolecular compounds.

The compound of Formula (I) is a TLR7 inhibitor and has the structure: 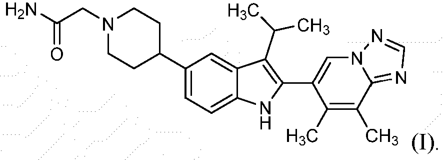 The chemical name for the compound of Formula (I) is 2-(4-(2-(7,8-dimethyl-[1,2,4]triazolo[1,5-a]pyridin-6-yl)-3-isopropyl-1H-indol-5-yl)piperidin-1-yl)acetamide. The discovery and synthesis of the compound of Formula (I) is described in WO 2018/005586 A1.

Prednisolone is a corticosteroid drug having the chemical name: 11,17-dihydroxy-17-(2-hydroxyacetyl)-10,13-dimethyl-6,7,8,9,10,11,12,13,14,15,16,17-dodecahydrocyclopenta[a] phenanthren-3-one. Prednisolone can be orally administered as a tablet, an orally disintegrating tablet, a solution, or a suspension. Ophthalmic prednisolone is available as a solution (liquid) and a suspension (eye drops). Prednisolone is available as a sodium salt. Prednisolone can also be administered as the prodrug prednisone. As used herein "prednisolone" includes both prednisolone and prednisone. As used herein "Pred" refers to prednisolone.

Hydroxychloroquine has the structure 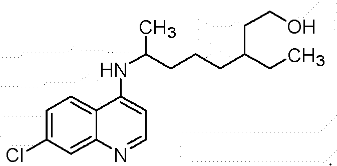 As used herein "HCQ" refers to hydroxychloroquine. Hydroxychloroquine is used to treat or prevent auto-immune diseases including lupus and rheumatoid arthritis. It can reduce skin problems in lupus, including cutaneous lupus erythematosus and prevent swelling/pain in arthritis. Hydroxychloroquine is available as a sulfate salt in which 200 mg of the sulfate salt is equal to 155 mg of the base.

Unless otherwise defined, scientific and technical terms used in connection with the present disclosure and invention shall have the meanings that are commonly understood by those of ordinary skill in the art. Further, unless otherwise required by context, singular terms shall include pluralities and plural terms shall include the singular.

The terms "treat," "treating," and "treatment," as used herein, refer to any type of intervention or process performed on, or administering an active agent to, the patient with the objective of reversing, alleviating, ameliorating, inhibiting, or slowing down or preventing the progression, development, severity or recurrence of a symptom, complication, condition or biochemical indicia associated with a disease. Treatment includes therapeutic treatment and prophylactic or preventative measures, wherein the object is prevent or lessen the targeted condition or disorder.

The term "therapeutically effective amount" or "therapeutically effective dosage" of a drug or therapeutic agent refers to an amount of a drug effective to treat a disease or disorder in a patient. In certain embodiments, an effective amount refers to an amount effective, at dosages and for period of time necessary, achieve the desired therapeutic or prophylactic result. The ability of a therapeutic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

Therapeutically effective amounts of a TLR7 inhibitor, prednisolone, and hydroxychloroquine may vary according to factors such as the disease state, age, sex, and weight of the patient, and abilities of the TLR7 inhibitor, prednisolone, and hydroxychloroquine to elicit a desired response in the patient. Therapeutically effective amounts of the TLR7 inhibitor, prednisolone, and hydroxychloroquine encompasses an amount in which any toxic or detrimental effects of the TLR7 inhibitor, prednisolone, and hydroxychloroquine are outweighed by the therapeutically beneficial effects.

The terms "administering" and "administration" refers to the physical introduction of a composition comprising a therapeutic agent to a patient, using any of the various methods and delivery systems known to those skilled in the art. Routes of administration for the TLR7 inhibitor and the second agent include enteral, topical, and mucosal administration such as oral, topical, sublingual, rectal, intranasal, and intravenous administration, and parenteral administration such as intravenous, intramuscular, and subcutaneous injection.

Administration "in combination with" one or more further therapeutic agents includes simultaneous (concurrent) and consecutive (sequential) administration in any order. For example, the patient may swallow the oral dosage form of the TLR7 inhibitor and the oral dosage form for the second agent in either order (consecutive); or may swallow both oral dosage forms together (concurrent).

The term "patient" includes human and other mammalian subjects that receive therapeutic treatment.

### DETAILED DESCRIPTION

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. Embodiments identified herein as exemplary or preferred are intended to be illustrative and not limiting.

References herein to methods of treatment using one or more active substances should be understood to relate to said substance(s) for use in the method of treatment.

Described herein are one or more methods of treating a patient having cutaneous lupus erythematosus.

One instance of the disclosure provides a method of treating a patient having cutaneous lupus erythematosus, comprising administering to said patient a therapeutically effective dose of a TLR7 inhibitor or a pharmaceutically acceptable salt thereof, in combination with a therapeutically effective dose of a TLR7 inhibitor or a pharmaceutically acceptable salt thereof; and a therapeutically effective dose of a second agent selected from prednisolone and hydroxychloroquine, or a pharmaceutically acceptable salt thereof. The present invention relates to the method in which the TLR7 inhibitor is the compound of Formula (I).

One instance of the disclosure provides a method of treating a patient having cutaneous lupus erythematosus, comprising administering to said patient a therapeutically effective dose of a TLR7 inhibitor or a pharmaceutically acceptable salt thereof, in combination with a therapeutically effective dose of prednisolone or a pharmaceutically acceptable salt thereof. The present invention relates to the method in which the TLR7 inhibitor is the compound of Formula (I).

One instance of the disclosure provides a method of treating a patient having cutaneous lupus erythematosus, comprising administering to said patient a therapeutically effective dose of a TLR7 inhibitor or a pharmaceutically acceptable salt thereof, in combination with a therapeutically effective dose of hydroxychloroquine or a pharmaceutically acceptable salt thereof. The present invention relates to the method in which the TLR7 inhibitor is the compound of Formula (I).

In one embodiment, said second agent is administered simultaneously with said TLR7 inhibitor. Included in this embodiment is a method in which the TLR7 inhibitor is the compound of Formula (I). In one embodiment, the second agent is selected from prednisolone or a pharmaceutically acceptable salt thereof, wherein said second agent is administered simultaneously with said TLR7 inhibitor. In one embodiment, the second agent is selected from hydroxychloroquine, or a pharmaceutically acceptable salt thereof, wherein said second agent is administered simultaneously with said TLR7 inhibitor.

In one embodiment, the second agent is selected from prednisolone and hydroxychloroquine, or a pharmaceutically acceptable salt thereof, wherein said second agent is administered sequentially with said TLR7 inhibitor. Included in this embodiment is a method in which the TLR7 inhibitor is administered prior to the administration of the second agent. Also included in this embodiment is a method in which the TLR7 agent is administered after the second agent.

In one embodiment, the second agent is selected from prednisolone or a pharmaceutically acceptable salt thereof, wherein said second agent is administered sequentially with said TLR7 inhibitor. Included in this embodiment is a method in which the TLR7 inhibitor is administered prior to the administration of the second agent. Also included in this embodiment is a method in which the TLR7 agent is administered after the second agent.

In one embodiment, the second agent is selected from hydroxychloroquine or a pharmaceutically acceptable salt thereof, wherein said second agent is administered sequentially with said TLR7 inhibitor. Included in this embodiment is a method in which the TLR7 inhibitor is administered prior to the administration of the second agent. Also included in this embodiment is a method in which the TLR7 agent is administered after the second agent.

In one embodiment, a therapeutically effective dose of the compound of Formula (I) is in the range of 0.1 to 100 mg.

In one embodiment, a therapeutically effective dose of the compound of prednisolone is in the range of 0.5 to 50 mg.

In one embodiment, a therapeutically effective dose of the compound of hydroxychloroquine is in the range of 1 to 20 mg.

The therapeutically effective dose of the TL7 inhibitor can be administered as a single daily dose (q.d.), divided and administered twice daily (b.i.d.), or divided and administered as three or more doses per day.

The therapeutically effective dose of prednisolone can be administered as a single daily dose (q.d.), divided and administered twice daily (b.i.d.), or divided and administered as three or more doses per day.

The therapeutically effective dose of hydroxychloroquine can be administered as a single daily dose (q.d.), divided and administered twice daily (b.i.d.), or divided and administered as three or more doses per day.

The therapeutically effective dose of the TLR7 inhibitor can be administered as a single daily dose.

In one embodiment, the therapeutically effective dose of the compound of Formula (I) is administered as a single daily dose.

In one embodiment, the therapeutically effective dose of prednisolone is administered as a single daily dose.

In one embodiment, the therapeutically effective dose of hydroxychloroquine is administered as a single daily dose.

In one embodiment, the therapeutically effective dose of the TLR7 inhibitor is administered as a single daily dose and the therapeutically effective dose of the second agent selected from prednisolone and hydroxychloroquine is administered as a single dose. Included in this embodiment, is a method in which the TLR7 inhibitor and the second agent are administered once a day, wherein the TLR7 inhibitor and the second agent are administered simultaneously, or the TLR7 inhibitor is administered immediately before or after the administration of the second agent. Also include in this embodiment is a method in which the TLR7 inhibitor is the compound of Formula (I). Additionally, included in this embodiment is a method in which the second agent is prednisolone. Further, included in this embodiment is a method in which the second agent is hydroxychloroquine.

In one embodiment, the therapeutically effective dose of the TLR7 inhibitor is administered as a twice daily dose and the therapeutically effective dose of the second agent is administered either one or twice daily dose. Included in this embodiment is a method in which the TLR7 inhibitor is the compound of Formula (I).

In one embodiment, the therapeutically effective dose of the TLR7 inhibitor is administered as a twice daily dose and the therapeutically effective dose of the second agent selected from prednisolone and hydroxychloroquine is administered as a twice dose. Included in this embodiment, is a method in which the TLR7 inhibitor and the second agent are administered twice a day, wherein the TLR7 inhibitor and the second agent are administered simultaneously twice daily, or the TLR7 inhibitor is administered immediately before or after the administration of the second agent twice daily. Also include in this embodiment is a method in which the TLR7 inhibitor is the compound of Formula (I). Additionally, included in this embodiment is a method in which the second agent is prednisolone. Further, included in this embodiment is a method in which the second agent is hydroxychloroquine.

In an another embodiment, a therapeutically effective dose of the TLR7 inhibitor or a pharmaceutically acceptable salt thereof is administered to said patient in combination with a therapeutically effective dose of a second agent selected from prednisolone and hydroxychloroquine, or a pharmaceutically acceptable salt thereof, and in combination with one or more addition third agents. Examples of suitable third agents include corticosteroids, rolipram, calphostin, cytokine-suppressive anti-inflammatory drugs (CSAIDs), Interleukin-10, glucocorticoids, salicylates, nitric oxide, and other immunosuppressants; nuclear translocation inhibitors, such as deoxyspergualin (DSG); nonsteroidal anti-inflammatory drugs (NSAIDs) such as ibuprofen, celecoxib and rofecoxib; steroids such as dexamethasone; antiproliferative agents such as methotrexate, leflunomide, FK506 (tacrolimus, PROGRAF^{®}); cytotoxic drugs such as azathiprine and cyclophosphamide; TNF-α inhibitors such as tenidap, anti-TNF antibodies or soluble TNF receptor, and rapamycin (sirolimus or RAPAMUNE^{®}) or derivatives thereof. The above third agents, when employed in combination with the combinations of Compound (I) and the second agent, may be used, for example, in those amounts indicated in the *Physicians' Desk Reference* (PDR) or as otherwise determined by one of ordinary skill in the art. The one or more third agents may be administered prior to, simultaneously with, or following the administration of Compound (I) or the second agent.

### EXAMPLES

### Cutaneous Lupus Erythematosus Study in Mice

### Materials and Methods

All the animal experimental procedures were reviewed and approved by the Institutional Animal Ethics Committee (IAEC) and conducted in accordance with procedures set by the Committee For The Purpose of Control and Supervision on Experiments on Animals (CPCSEA). Mice were group housed in Syngene Laboratory Animal Research Facility (SLAR, Bangalore India; AAALAC accredited), and maintained under normal 12 h light /12 h dark cycle with ad libitum access to food and water. At the end of the studies, animals were euthanized by CO₂ asphyxiation for plasma and tissue collection.

### Cutaneous Model of Lupus

Female MRL/lpr mice of 12 to 14 weeks of age were screened and randomized based on the titers of anti-dsDNA antibodies and urinary neutrophil gelatinase-associated lipocalin (NGAL). Mice were treated orally, once daily for 8 weeks with vehicle (10% ethanol; 45% PEG 300; 5% Pluronic F-68; 40% 20 mM citrate buffer) or different doses of test compound (Compound (I)) or reference (prednisolone) compound. The effect of vehicle, test or reference compounds on cutaneous disease severity were assessed by evaluating skin lesions on the neck, head, face and ear separately. The severity of skin lesions was assessed every week by counting the number of lesions as well as erythema (on the scale of 0 to 4, considering zero - none; 1 - mild, 2 - moderate, 3 -moderate to severe and 4 - very marked and severe). Total erythema was derived by adding the erythema scores of neck, head, ear and face lesions. Since female MRL/lpr mice also develop nephritis in addition to cutaneous lesions, the effect of vehicle, test or reference compounds on renal disease were evaluated by measuring proteinuria, urinary NGAL and autoantibody titers like anti-Ro, anti-smRNP and anti-dsDNA Ab titer. One week before termination of the experiment, mice were bled at various time points to capture the complete pharmacokinetic profile of the test or reference compound and also the extent of inhibition of gardiquimod-induced IL-6 in an ex vivo whole blood assay. Spleen samples were taken at the end of the study to measure the impact of treatment on IL-6 and IFNα-producing cell population by flow cytometry. Blood samples were analyzed for gene expression by qPCR and for TLR7 target coverage utilizing an ex vivo blood assay of gardiquimod induced IL-6 production. Statistical analysis was done using one-way ANOVA with Dunnett's test to calculate the significance of test or reference compound treated groups vs. vehicle control group. Percent reduction in disease severity was calculated for each parameter, for test or reference compound treated groups vs. vehicle control group.

### Inhibition of Cutaneous manifestations in MRL/lpr Model of Lupus

Female MRL/lpr mice develop cutaneous lesions in a spontaneous manner, making this a widely accepted preclinical murine model of cutaneous lupus. In addition to cutaneous manifestations, these mice also showed kidney injury. Compound was investigated for its effects on both skin lesions and on kidney injury markers in this model. In this study, mice were randomized based on urinary NGAL and anti-dsDNA Ab titer. Immediately after recruitment, mice were treated with Compound (I) and prednisolone for 8 weeks.

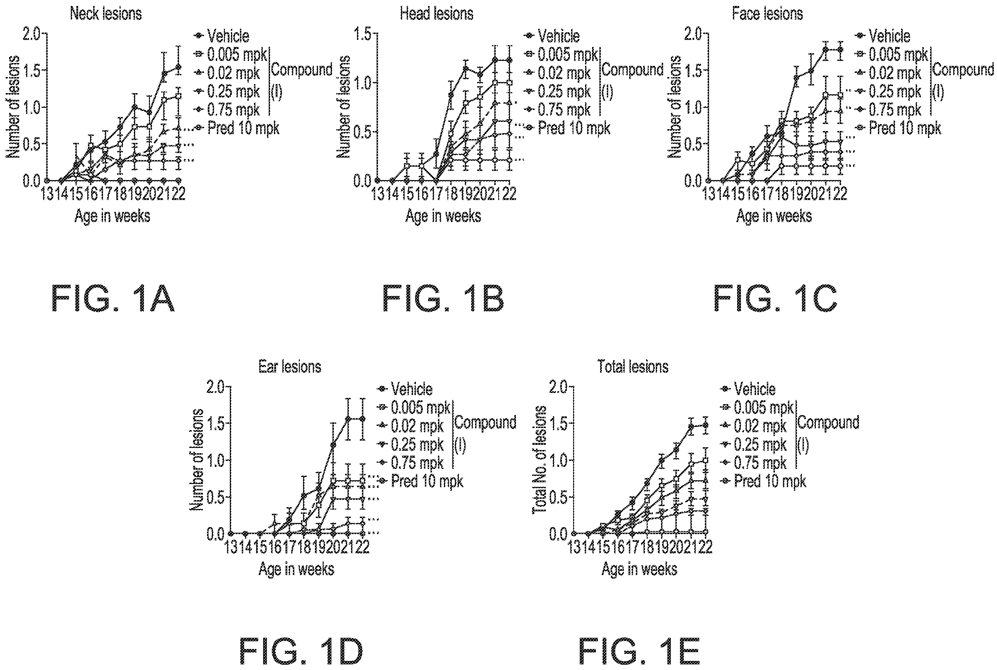

Figure 1: Inhibition of cutaneous lesions in female MRL/lpr mice following treatment with Compound (I). Mice were dosed orally for 8 weeks.. Skin lesions were then assessed on neck (A), head (B), face (C), and ear (D). The impact of treatment on the total lesions were also represented by adding the number of lesions present on face, neck, head, and ear. (E) Data are from one experiment with 13 to 15 mice per group. *P < 0.01, **P < 0.001, ***P < 0.0001 versus vehicle by two-way ANOVA with a Bonferroni test.

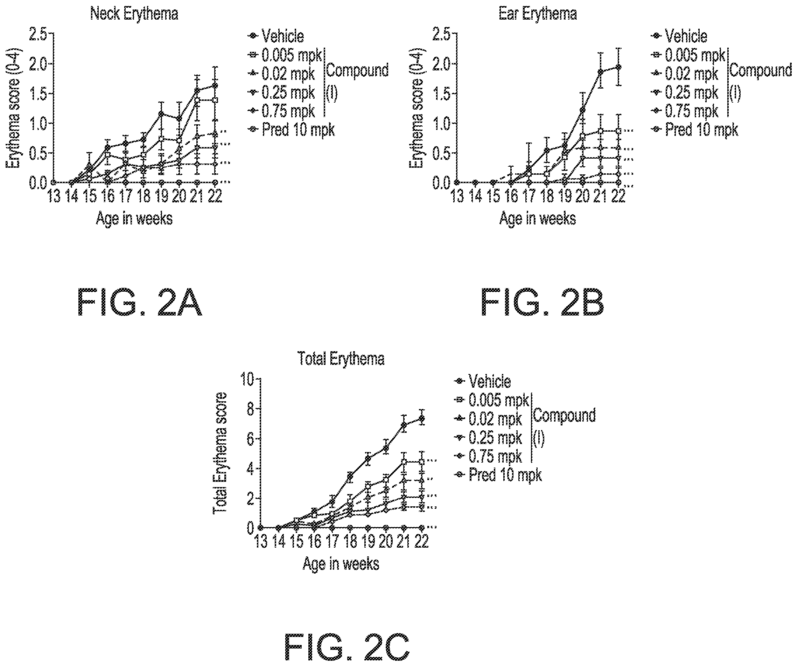

Figure 2: Treatment of female MRL/lpr mice with Compound (I) suppressed erythema of cutaneous lesions. Mice were dosed orally for 8 weeks. Erythema of skin lesions were assessed on neck (A) and ear (B). The impact of treatment on the total erythema was also represented by assessing the erythematous score of skin lesions present on back, face, neck, ear and head (C). Data are from one experiment with 13 to 15 mice per group. *P < 0.01, **P < 0.001, ***P < 0.0001 versus vehicle by two-way ANOVA with a Bonferroni test.

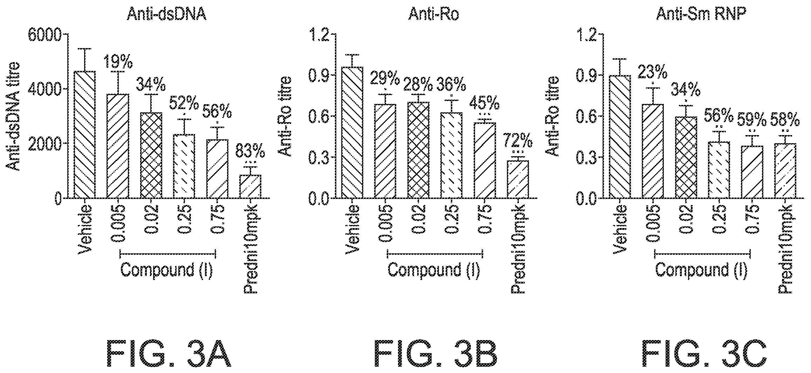

Figure 3: Inhibition of autoantibodies in female MRL/lpr mice treated with Compound (I). Mice were dosed orally for 8 weeks with respective treatment. Following 8 weeks of dosing, anti-dsDNA (A) and anti-Ro (B) and anti-smRNP antibody titers (C) were assessed. Data are from one experiment with 13 to 15 mice per group. Percent inhibition relative to the vehicle group is shown above each bar. *P < 0.01, **P < 0.001, ***P < 0.0001 versus vehicle by one-way ANOVA with a Dunnett's test.

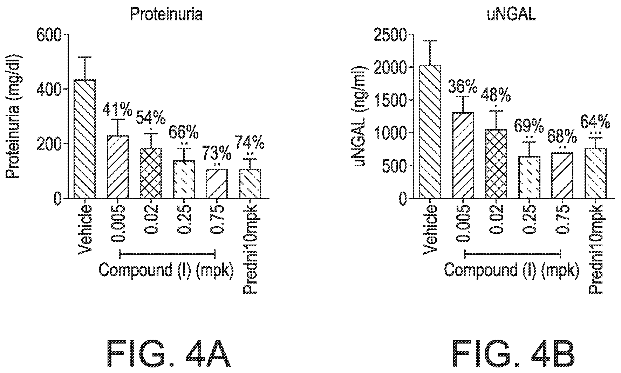

Figure 4: Inhibition of kidney injury markers in female MRL/lpr mice treated with Compound (I). Mice were dosed orally for 8 weeks with respective treatment. Following 8 weeks of dosing, proteinuria (A) and urinary NGAL (B) were assessed. Data are from one experiment with 13 to 15 mice per group. Percent inhibition relative to the vehicle group is shown above each bar. *P < 0.01, **P < 0.001, ***P < 0.0001 versus vehicle by one-way ANOVA with a Dunnett's test.

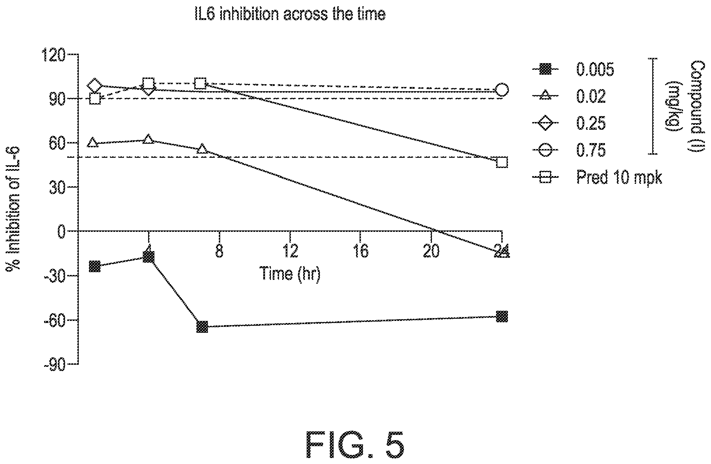

Figure 5: Compound (I) inhibited TLR7-dependent IL-6 induction in an ex vivo assay in female MRL/lpr model of lupus. Mice were dosed orally for 7 weeks with respective treatment. Following 7 weeks of dosing, mouse whole blood was drawn at different time points and challenged with gardiquimod for 16 to 18 hours. IL-6 inhibition was assessed by ELISA. Data are from one experiment in which measurements were made in three mice per group per time point.

Treatment with Compound (I) improved survival of mice in Female MRL/lpr model of lupus compared to vehicle

**TABLE 1**

| Treatment Groups | Dose (mg/kg) | % Survival |
|---|---|---|
| Vehicle | NA | 87 |
| Compound (I) | 0.005 | 87 |
| | 0.02 | 93 |
| | 0.25 | 100 |
| | 0.75 | 100 |
| Prednisolone | 10 | 100 |

| | | |
|---|---|---|
| Abbreviation: NA, not applicable. | | |

The data in Table 1 shows that treatment with Compound (I) improved survival of the mice in this study. Whereas only 87% of vehicle mice survived for 8 weeks, mice receiving doses of 0.25 and 0.75 mg/kg of Compound (I) achieved 100% survival.

As shown in Figure 1, Compound (I) showed significant and robust dose-dependent suppression of cutaneous manifestations as assessed by measuring the number of lesions on face, neck, head, and ear. The impact of treatment on the total lesions were also represented by adding the number of lesions present on face, neck, head, and ear. Furthermore, the effect of Compound (I) was also evaluated by assessing the erythema on neck and ear and represented individually. Additionally, skin lesions were also evaluated for total erythema after assessing the erythema of skin lesions present on back, face, neck, ear and head. A significant reduction in the erythema upon treatment with Compound (I) was observed Figure 2. Representative images of lesions in mice treated with Compound (I) and vehicle mice at the 8 week time point are shown in Figure 3.

Dose-dependent inhibition of cutaneous lesions including number of lesions as well as erythema of lesions was observed with treatment with Compound (I) in mice. Greater efficacy was observed with 0.25 mg/kg and 0.75 mg/kg doses of Compound (I). These two doses resulted in improved survival, 100% versus 87% survival of vehicle treated mice. At these doses, Compound (I) showed suppression of lupus nephritis as assessed by kidney injury markers like proteinuria and urinary NGAL. Additionally, at these two doses, Compound (I) showed significant suppression of anti-dsDNA, anti-RO and anti-smRNP auto-antibody titers and inhibited Type I IFN response gene expression.

### Combination Studies

**TABLE 2**

| No. | Treatment | Dose (mg/kg) | n |
|---|---|---|---|
| 1 | Vehicle | 5ml/kg, qd | 15 |
| 2 | Compound (I) | 0.25 | 15 |
| 3 | Prednisolone | 1 | 15 |
| 4 | HCQ | 100 | 15 |
| 5 | Compound (I) + Prednisolone | 0.25 + 1 | 15 |
| 6 | Compound (I) + HCQ | 0.25 + 100 | 15 |
| 7 | Prednisolone | 10 | 15 |

### 1. Combination Studies of Compound (I) with Prednisolone and Compound (I) with hydroxychloroquine (HQC) in MRL/lpr Model of Lupus - Established Disease

Female MRL/lpr mice of 15 weeks of age were treated for 5 weeks. Mice were recruited when they showed group average of total skin lesions score as 1.5 to 2. Total skin lesions score includes cumulative score of Neck/Back, Face, Head and Ear lesions

**TABLE 3**

| No. | Treatment | Dose (mg/kg) | % Survival |
|---|---|---|---|
| 1 | Vehicle | 5ml/kg, qd | 93.3 |
| 2 | Compound (I) | 0.25 | 100 |
| 3 | Prednisolone | 1 | 93.3 |
| 4 | HCQ | 100 | 93.3 |
| 5 | Compound (I) + Prednisolone | 0.25 + 1 | 100 |
| 6 | Compound (I) + HCQ | 0.25 + 100 | 100 |
| 7 | Prednisolone | 10 | 93.3 |

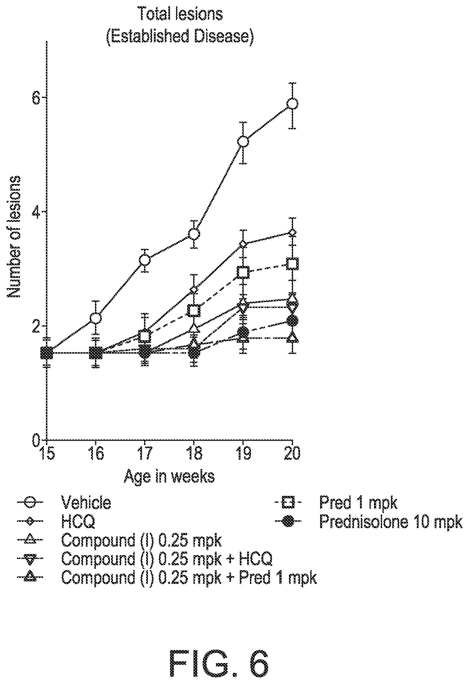

Figure 6: Treatment with Compound (I) suppressed the total number of cutaneous lesions in mice in established disease compared to treatment with vehicle. Monotherapy with prednisolone and hydroxychloroquine showed suppression of cutaneous lesions but less than treatment with Compound (I). Treatment with the combination of Compound (I) + prednisolone and the combination of Compound (I) and hydroxychloroquine showed increased suppression compared to hydroxychloroquine and 1 mpk prednisolone monotherapies.

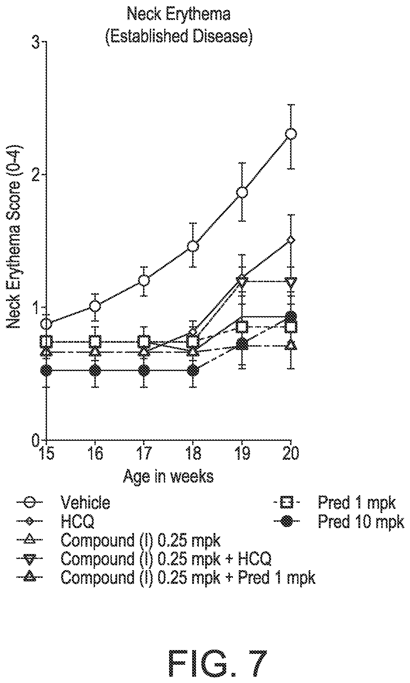

Figure 7: Treatment with Compound (I) suppressed erythema of cutaneous lesions on the neck of mice in established disease compared to vehicle. Monotherapy with prednisolone and hydroxychloroquine showed suppression of erythema of cutaneous lesions on the neck of mice. Treatment with the combination of Compound (I) + prednisolone and the combination of Compound (I) and hydroxychloroquine showed suppression of erythema of cutaneous lesions on the neck of mice.

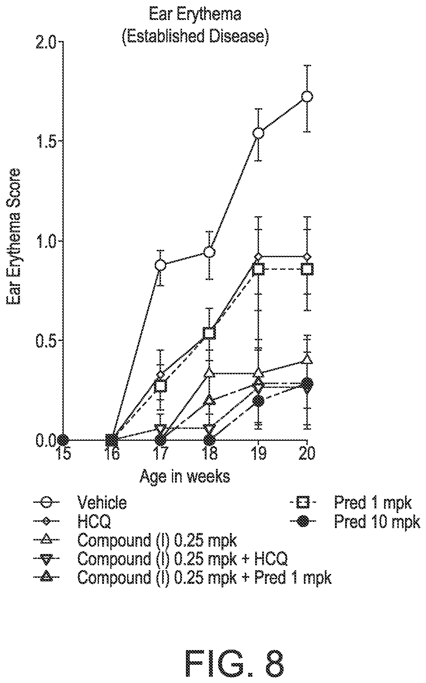

Figure 8: Treatment with Compound (I) suppressed erythema of cutaneous lesions on the ear of mice in established disease compared to vehicle. Monotherapy with prednisolone and hydroxychloroquine showed suppression of erythema of cutaneous lesions on the ear of mice but less than treatment with Compound (I). Treatment with the combination of Compound (I) + prednisolone and the combination of Compound (I) and hydroxychloroquine showed increased suppression of erythema of cutaneous lesions compared to hydroxychloroquine and 1 mpk prednisolone monotherapies.

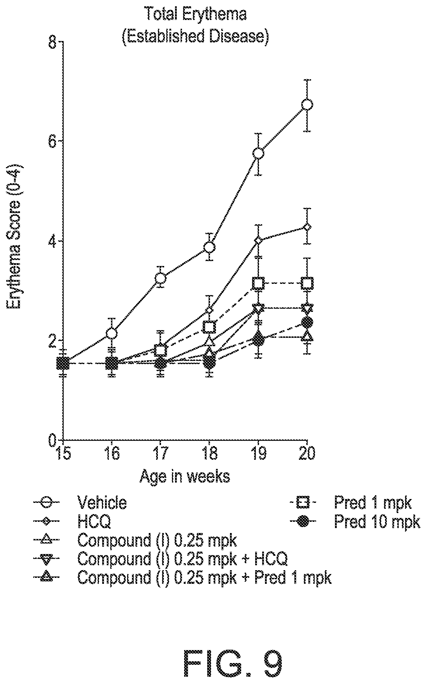

Figure 9: Treatment with Compound (I) suppressed total erythema of cutaneous lesions on mice in established disease compared to vehicle. Monotherapy with prednisolone and hydroxychloroquine showed suppression of total erythema of cutaneous lesions on mice. Treatment with the combination of Compound (I) + prednisolone and the combination of Compound (I) and hydroxychloroquine showed suppression of total erythema of cutaneous lesions on mice.

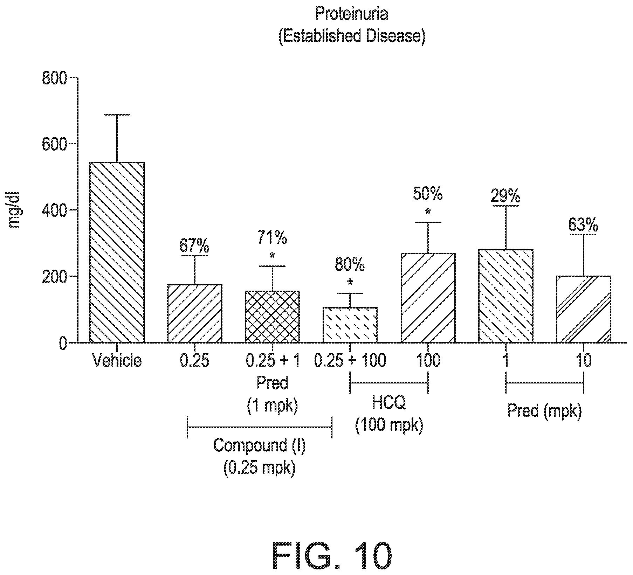

Figure 10: Treatment with Compound (I) showed suppression of the kidney injury marker proteinuria and urinary neutrophill gelatinase-associated lipocalin (NGAL) in established disease. Monotherapy with prednisolone or hydroxychloroquine showed suppression of the kidney injury marker proteinuria. The combination of Compound (I) with Prednisolone and the combination of Compound (I) with hydroxychloroquine showed increased suppression of the kidney injury marker proteinuria.

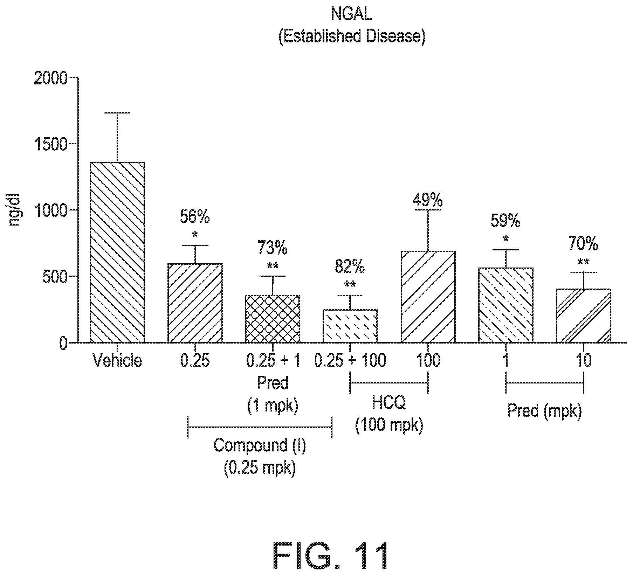

Figure 11: Treatment with Compound (I) showed suppression of the kidney injury marker urinary NGAL in established disease. Monotherapy with prednisolone or hydroxychloroquine showed suppression of the kidney injury marker urinary NGAL. The combination of Compound (I) with Prednisolone and the combination of Compound (I) with hydroxychloroquine showed increased suppression of the kidney injury marker urinary NGAL.

### 2. Combination Studies of Compound (I) with Prednisolone and Compound (I) with hydroxychloroquine (HQC) in MRL/lpr Model of Lupus - Advanced Disease

Female MRL/lpr mice of 16 weeks of age were treated for 5 weeks. Mice were recruited when they showed group average of total skin lesions score as 2 to 2.5. Total skin lesions score includes cumulative score of Neck/Back, Face, Head and Ear lesions. Table 2 shows the treatments, doses, and number of mice in each treatment group.

**TABLE 4**

| No. | Treatment | Dose (mg/kg) | % Survival |
|---|---|---|---|
| 1 | Vehicle | 5mL/kg, qd | 80 |
| 2 | Compound (I) | 0.25 | 93.3 |
| 3 | Prednisolone | 1 | 86.6 |
| 4 | HCQ | 100 | 100 |
| 5 | Compound (I) + Prednisolone | 0.25 + 1 | 100 |
| 6 | Compound (I) + HCQ | 0.25 + 100 | 100 |
| 7 | Prednisolone | 10 | 93.3 |

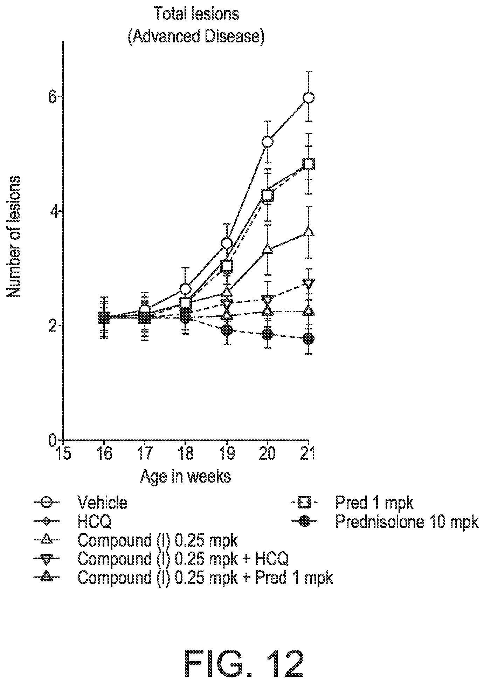

Figure 12: Treatment with Compound (I) suppressed the total number of cutaneous lesions in mice in advanced disease compared to treatment with vehicle. Monotherapy with prednisolone and hydroxychloroquine showed suppression of cutaneous lesions but less than treatment with Compound (I). Treatment with the combination of Compound (I) + prednisolone and the combination of Compound (I) and hydroxychloroquine showed increased suppression compared to hydroxychloroquine and 1 mpk prednisolone monotherapies.

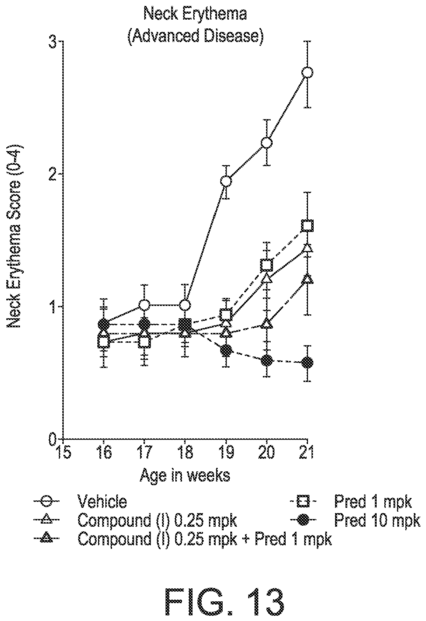

Figure 13: Treatment with Compound (I) suppressed erythema of cutaneous lesions on the neck of mice in advanced disease compared to vehicle. Monotherapy with prednisolone showed suppression of erythema of cutaneous lesions on the neck of mice. Treatment with the combination of Compound (I) + prednisolone showed suppression of erythema of cutaneous lesions on the neck of mice.

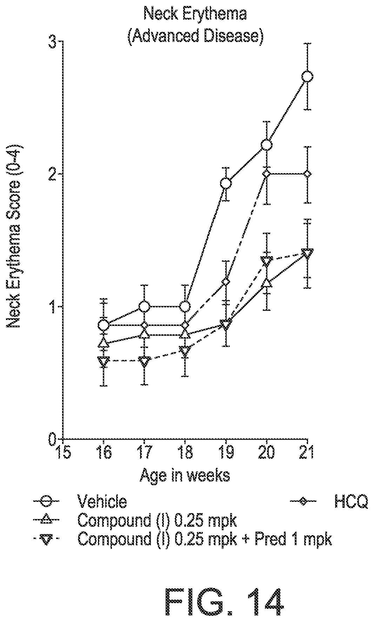

Figure 14: Treatment with Compound (I) suppressed erythema of cutaneous lesions on the neck of mice in advanced disease compared to vehicle. Monotherapy with hydroxychloroquine showed suppression of erythema of cutaneous lesions on the neck of mice. Treatment with the Compound (I) and treatment with the combination of Compound (I) + hydroxychloroquine showed increased suppression of erythema of cutaneous lesions on the neck of mice compared the treatment with hydroxychloroquine.

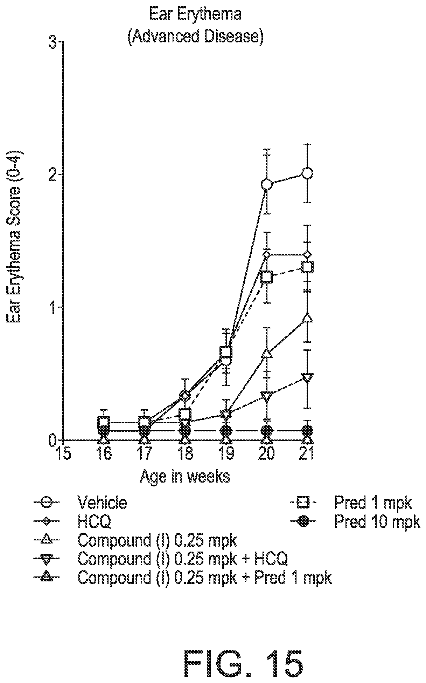

Figure 15: Treatment with Compound (I) suppressed erythema of cutaneous lesions on the ear of mice in advanced disease compared to vehicle. Monotherapy with prednisolone and hydroxychloroquine showed suppression of erythema of cutaneous lesions on the ear of mice but less than treatment with Compound (I). Treatment with the combination of Compound (I) + prednisolone and the combination of Compound (I) and hydroxychloroquine showed increased suppression of erythema of cutaneous lesions compared to hydroxychloroquine and 1 mpk prednisolone monotherapies.

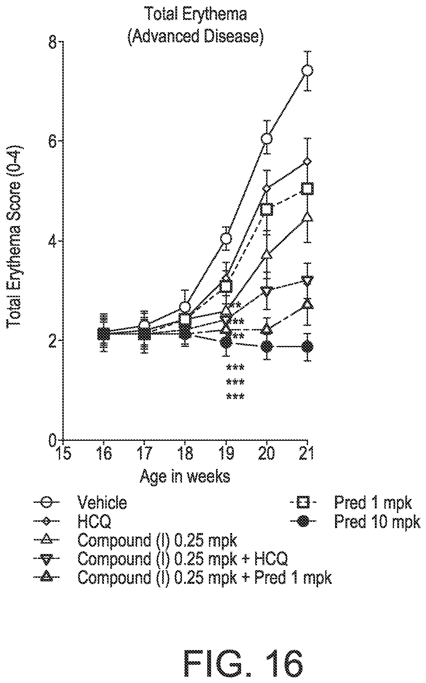

Figure 16: Treatment with Compound (I) suppressed total erythema of cutaneous lesions on mice in advanced disease compared to vehicle. Monotherapy with prednisolone and hydroxychloroquine showed suppression of total erythema of cutaneous lesions on mice. Treatment with the combination of Compound (I) + prednisolone and the combination of Compound (I) and hydroxychloroquine showed suppression of total erythema of cutaneous lesions on mice compared to hydroxychloroquine and 1 mpk prednisolone monotherapies.

## Claims

1. A TLR7 inhibitor or a pharmaceutically acceptable salt thereof for use in the treatment of cutaneous lupus erythematosus comprising administering a therapeutically effective dose of said TLR7 inhibitor or pharmaceutically acceptable salt thereof, in combination with a therapeutically effective dose of a second agent selected from prednisolone and hydroxychloroquine, or a pharmaceutically acceptable salt thereof; wherein said TLR7 inhibitor is: 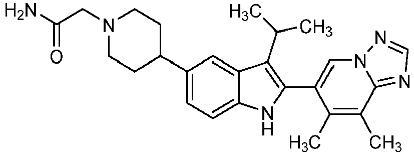 or a pharmaceutically acceptable salt thereof.

2. The TLR7 inhibitor or pharmaceutically acceptable salt thereof for use according to claim 1, the method comprising administering to said patient a therapeutically effective dose of said TLR7 inhibitor or pharmaceutically acceptable salt thereof, in combination with a therapeutically effective dose of prednisolone or a pharmaceutically acceptable salt thereof.

3. The TLR7 inhibitor or pharmaceutically acceptable salt thereof for use according to claim 1, the method comprising administering to said patient a therapeutically effective dose of said TLR7 inhibitor or pharmaceutically acceptable salt thereof, in combination with a therapeutically effective dose of hydroxychloroquine or a pharmaceutically acceptable salt thereof.

4. The TLR7 inhibitor or pharmaceutically acceptable salt thereof for use according to claim 1, wherein said therapeutically effective dose of said TLR7 inhibitor is from 0.1 to 100 mg per day.

5. The TLR7 inhibitor or pharmaceutically acceptable salt thereof for use according to claim 1, wherein said therapeutically effective dose of said prednisolone is 0.5 to 50 mg per day.

6. The TLR7 inhibitor or pharmaceutically acceptable salt thereof for use according to claim 1, wherein said therapeutically effective dose of said hydroxychloroquine is 1 to 20 mg per day.

7. The TLR7 inhibitor or pharmaceutically acceptable salt thereof for use according to claim 1, wherein said TLR7 inhibitor and said second agent are administered concurrently.

8. The TLR7 inhibitor or pharmaceutically acceptable salt thereof for use according to claim 1, wherein said TLR7 inhibitor and said second agent are administered sequentially.

9. The TLR7 inhibitor or pharmaceutically acceptable salt thereof for use according to claim 1, wherein said TLR7 inhibitor is administered prior to administration of said second agent.

10. The TLR7 inhibitor or pharmaceutically acceptable salt thereof for use according to claim 1, wherein said second agent is administered prior to administration of said TLR7 inhibitor.

11. A TLR7 inhibitor or a pharmaceutically acceptable salt thereof, in combination with a second agent selected from prednisolone, or a pharmaceutically acceptable salt thereof, for use in the treatment of cutaneous lupus erythematosus, wherein said TLR7 inhibitor is:

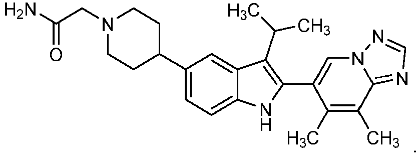

12. A TLR7 inhibitor or a pharmaceutically acceptable salt thereof, in combination with a second agent selected from hydroxy chloroquine, or a pharmaceutically acceptable salt thereof, for use in the treatment of cutaneous lupus erythematosus, wherein said TLR7 inhibitor is:

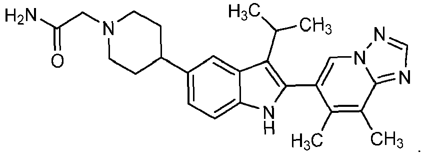

## Patentansprüche

1. Ein TLR7-Inhibitor oder ein pharmazeutisch verträgliches Salz davon zur Verwendung bei der Behandlung von kutanem Lupus erythematodes, umfassend die Verabreichung einer therapeutisch wirksamen Dosis des TLR7-Inhibitors oder eines pharmazeutisch verträglichen Salzes davon in Kombination mit einer therapeutisch wirksamen Dosis eines zweiten Wirkstoffs, ausgewählt aus Prednisolon und Hydroxychloroquin, oder eines pharmazeutisch verträglichen Salzes davon; wobei der TLR7-Inhibitor 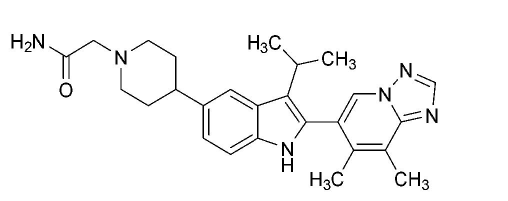 oder ein pharmazeutisch verträgliches Salz davon ist.

2. Der TLR7-Inhibitor oder ein pharmazeutisch verträgliches Salz davon zur Verwendung nach Anspruch 1, wobei das Verfahren das Verabreichen einer therapeutisch wirksamen Dosis des TLR7-Inhibitors oder eines pharmazeutisch verträglichen Salzes davon an den Patienten in Kombination mit einer therapeutisch wirksamen Dosis von Prednisolon oder einem pharmazeutisch verträglichen Salz davon umfasst.

3. Der TLR7-Inhibitor oder ein pharmazeutisch verträgliches Salz davon zur Verwendung nach Anspruch 1, wobei das Verfahren das Verabreichen einer therapeutisch wirksamen Dosis des TLR7-Inhibitors oder eines pharmazeutisch verträglichen Salzes davon an den Patienten in Kombination mit einer therapeutisch wirksamen Dosis von Hydroxychloroquin oder einem pharmazeutisch verträglichen Salz davon umfasst.

4. Der TLR7-Inhibitor oder ein pharmazeutisch verträgliches Salz davon zur Verwendung nach Anspruch 1, wobei die therapeutisch wirksame Dosis des TLR7-Inhibitors 0,1 bis 100 mg pro Tag beträgt.

5. Der TLR7-Inhibitor oder ein pharmazeutisch verträgliches Salz davon zur Verwendung nach Anspruch 1, wobei die therapeutisch wirksame Dosis des Prednisolons 0,5 bis 50 mg pro Tag beträgt.

6. Der TLR7-Inhibitor oder ein pharmazeutisch verträgliches Salz davon zur Verwendung nach Anspruch 1, wobei die therapeutisch wirksame Dosis des Hydroxychloroquins 1 bis 20 mg pro Tag beträgt.

7. Der TLR7-Inhibitor oder ein pharmazeutisch verträgliches Salz davon zur Verwendung nach Anspruch 1, wobei der TLR7-Inhibitor und der zweite Wirkstoff gleichzeitig verabreicht werden.

8. Der TLR7-Inhibitor oder ein pharmazeutisch verträgliches Salz davon zur Verwendung nach Anspruch 1, wobei der TLR7-Inhibitor und der zweite Wirkstoff hintereinander verabreicht werden.

9. Der TLR7-Inhibitor oder ein pharmazeutisch verträgliches Salz davon zur Verwendung nach Anspruch 1, wobei der TLR7-Inhibitor vor Verabreichung des zweiten Wirkstoffs verabreicht wird.

10. Der TLR7-Inhibitor oder ein pharmazeutisch verträgliches Salz davon zur Verwendung nach Anspruch 1, wobei der zweite Wirkstoff vor Verabreichung des TLR7-Inhibitors verabreicht wird.

11. Ein TLR7-Inhibitor oder ein pharmazeutisch verträgliches Salz davon in Kombination mit einem zweiten Wirkstoff, ausgewählt aus Prednisolon oder einem pharmazeutisch verträglichen Salz davon, zur Verwendung bei der Behandlung von kutanem Lupus erythematodes, wobei der TLR7-Inhibitor

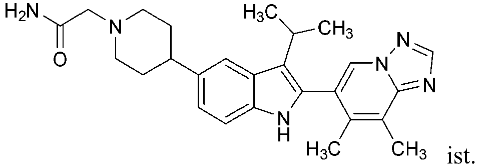

12. Ein TLR7-Inhibitor oder ein pharmazeutisch verträgliches Salz davon in Kombination mit einem zweiten Wirkstoff, ausgewählt aus Hydroxychloroquin oder einem pharmazeutisch verträglichen Salz davon, zur Verwendung bei der Behandlung von kutanem Lupus erythematodes, wobei der TLR7-Inhibitor

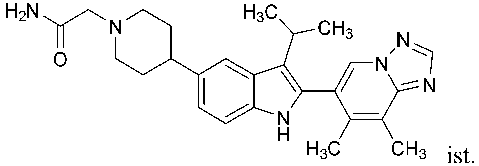

## Revendications

1. Inhibiteur de TLR7 ou un sel pharmaceutiquement acceptable de celui-ci destiné à être utilisé dans le traitement du lupus érythémateux cutané, comprenant l'administration d'une dose thérapeutiquement efficace dudit inhibiteur de TLR7 ou sel pharmaceutiquement acceptable de celui-ci, en combinaison avec une dose thérapeutiquement efficace d'un deuxième agent choisi parmi la prednisolone et l'hydroxychloroquine, ou d'un sel pharmaceutiquement acceptable de celles-ci, dans lequel ledit inhibiteur de TLR7 est : 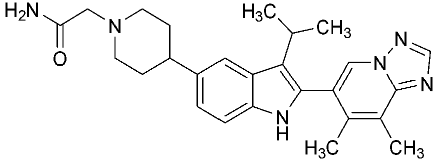 ou un sel pharmaceutiquement acceptable de celui-ci.

2. Inhibiteur de TLR7 ou sel pharmaceutiquement acceptable de celui-ci destiné à être utilisé selon la revendication 1, le procédé comprenant l'administration audit patient d'une dose thérapeutiquement efficace dudit inhibiteur de TLR7 ou sel pharmaceutiquement acceptable de celui-ci, en combinaison avec une dose thérapeutiquement efficace de prednisolone ou d'un sel pharmaceutiquement acceptable de celle-ci.

3. Inhibiteur de TLR7 ou sel pharmaceutiquement acceptable de celui-ci destiné à être utilisé selon la revendication 1, le procédé comprenant l'administration audit patient d'une dose thérapeutiquement efficace dudit inhibiteur de TLR7 ou sel pharmaceutiquement acceptable de celui-ci, en combinaison avec une dose thérapeutiquement efficace d'hydroxychloroquine ou d'un sel pharmaceutiquement acceptable de celle-ci.

4. Inhibiteur de TLR7 ou sel pharmaceutiquement acceptable de celui-ci destiné à être utilisé selon la revendication 1, dans lequel ladite dose thérapeutiquement efficace dudit inhibiteur de TLR7 est de 0,1 à 100 mg par jour.

5. Inhibiteur de TLR7 ou sel pharmaceutiquement acceptable de celui-ci destiné à être utilisé selon la revendication 1, dans lequel ladite dose thérapeutiquement efficace de ladite prednisolone est 0,5 à 50 mg par jour.

6. Inhibiteur de TLR7 ou sel pharmaceutiquement acceptable de celui-ci destiné à être utilisé selon la revendication 1, dans lequel ladite dose thérapeutiquement efficace de ladite hydroxychloroquine est 1 à 20 mg par jour.

7. Inhibiteur de TLR7 ou sel pharmaceutiquement acceptable de celui-ci destiné à être utilisé selon la revendication 1, dans lequel ledit inhibiteur de TLR7 et ledit deuxième agent sont administrés de manière simultanée.

8. Inhibiteur de TLR7 ou sel pharmaceutiquement acceptable de celui-ci destiné à être utilisé selon la revendication 1, dans lequel ledit inhibiteur de TLR7 et ledit deuxième agent sont administrés de manière séquentielle.

9. Inhibiteur de TLR7 ou sel pharmaceutiquement acceptable de celui-ci destiné à être utilisé selon la revendication 1, dans lequel ledit inhibiteur de TLR7 est administré avant l'administration dudit deuxième agent.

10. Inhibiteur de TLR7 ou sel pharmaceutiquement acceptable de celui-ci destiné à être utilisé selon la revendication 1, dans lequel ledit deuxième agent est administré avant l'administration dudit inhibiteur de TLR7.

11. Inhibiteur de TLR7 ou sel pharmaceutiquement acceptable de celui-ci, en combinaison avec un deuxième agent choisi parmi la prednisolone, ou un sel pharmaceutiquement acceptable de celle-ci, destiné à être utilisé dans le traitement du lupus érythémateux cutané, dans lequel ledit inhibiteur de TLR7 est :

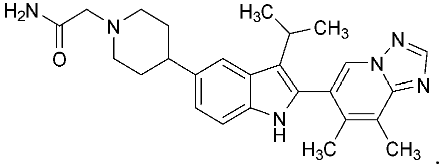

12. Inhibiteur de TLR7 ou sel pharmaceutiquement acceptable de celui-ci, en combinaison avec un deuxième agent choisi parmi l'hydroxychloroquine, ou un sel pharmaceutiquement acceptable de celle-ci, destiné à être utilisé dans le traitement du lupus érythémateux cutané, dans lequel ledit inhibiteur de TLR7 est :
